# EUROPÄISCHE PATENTSCHRIFT

(11) **EP 3 481 457 B1**
(45) Veröffentlichungstag und Bekanntmachung des Hinweises auf die Patenterteilung: **13.04.2022**
(21) Anmeldenummer: 17745251.3
(22) Anmeldetag: 07.07.2017
(51) Int. Cl.: A61B 5/00

(54) **VERFAHREN UND SYSTEM MIT SMARTPHONE ZUR ERFASSUNG VON SYMPTOMEN EINER PERITONITIS**
METHOD AND SYSTEM WITH A SMARTPHONE FOR DETECTING SYMPTOMS OF PERITONITIS
PROCÉDÉ ET SYSTÈME AVEC UN SMARTPHONE POUR DÉCELER DES SYMPTÔMES D'UNE PÉRITONITE

(30) Priorität: 07.07.2016 DE 102016008332
(43) Veröffentlichungstag der Anmeldung: 15.05.2019
(73) Patentinhaber: Fresenius Medical Care Deutschland GmbH, 61352 Bad Homburg (DE)
(72) Erfinder: WÜPPER, Andreas, 64572 Büttelborn (DE); MOISSL, Ulrich, 61184 Karben (DE)
(74) Vertreter: Herrmann, Uwe
(86) Internationale Anmeldenummer: PCT/EP2017/000803
(87) Internationale Veröffentlichungsnummer: WO 2018/007013

(56) Entgegenhaltungen:
- WO-A1-99/06082
- WO-A1-2012/087152
- WO-A1-2012/155067
- CN-U- 201 657 912
- TW-B- I 525 578
- US-A1- 2008 183 127
- US-A1- 2009 036 175

## Beschreibung

Die Erfindung betrifft ein Verfahren und ein System zur Erfassung von Symptomen einer Peritonitis.

Eine Peritonitis bzw. Bauchfellentzündung kann als Komplikation bei Peritonealdialyse-Patienten (PD-Patienten) auftreten und stellt eine ernste gesundheitliche Bedrohung dar. Es kann zu Versagen der Eignung des Peritoneums für die Dialyse (,technique failure') führen und eine Änderung der Behandlungsart auf Hämodialyse erforderlich machen.

Die Häufigkeit der mit Peritonealdialysen assoziierten Peritonitis liegt typischerweise bei etwa einem Ereignis pro Patient alle 20 - 38 Monate. Als Folge liegt die Todesrate bei ca. 3% und die ,technique failure' bei ca. 15%.

Eine Peritonealdialyse-Peritonitis (PD-Peritonitis) präsentiert sich durch eine trübe Drainagelösung, sogenannte ,cloudy effluent' (99%). Des Weiteren liegen Symptome vor wie Unterleibsschmerzen (95%), Übelkeit/Erbrechen (30%) oder Verstopfung/Durchfall (15%).

Als Therapie werden Antibiotika intraperitoneal und oral verabreicht. In der Regel wird bei Vorliegen von trüber Drainageflüssigkeit damit sofort begonnen, noch bevor Ergebnisse der Zellkultur vorliegen. Eine frühere Erkennung der Peritonitis ermöglicht einen früheren Behandlungsbeginn und damit bessere Aussichten auf Heilung und Begrenzung der Komplikationen.

Die Peritonealdialyse (PD) ist eine Heimtherapie, die vom Patienten selber ohne Anwesenheit eines Arztes oder einer Pflegekraft durchgeführt werden kann. Auf Zeichen einer Peritonitis muss der Patient selbst achten.

Aus WO 2012/155067 sind ein Verfahren und System zu Erfassung von Symptomen einer Peritonitis mittels einer Smartphone-Fotoaufnahme bekannt.

Vor diesem Hintergrund ist es Aufgabe der Erfindung, einem Patienten bei der Erkennung von Zeichen einer Peritonitis zu unterstützen.

Die Erfindung ist definiert durch die anhängigen unabhängigen Ansprüche.

Es können auch mehr als eine Fotoaufnahme ausgewertet werden. Wenn vorliegend von einer Fotoaufnahme die Rede ist, so soll darunter auch die Nutzung von mehr als einer Fotoaufnahme verstanden werden.

Die Auswertung kann darin bestehen, die von dem Patienten gemachten Fotos bzw. die von ihm eingegebenen Abfrageparameter in Bezug zu entsprechenden Referenzwerten von gesunden und von an einer Peritonitis erkrankten Patienten zu setzen.

Nach der Auswertung der Fotos und/oder des Abfrageparameters kann beispielsweise ein Hinweis an den Patienten ausgegeben werden, welcher ihm die Symptome einer Peritonitis erläutert und/oder es kann eine Angabe der Wahrscheinlichkeit des Vorliegens einer Peritonitis gemacht werden.

Die Wahrscheinlichkeit kann in Abhängigkeit von den erstellten Fotoaufnahmen bzw. von den eingegebenen Abfrageparametern berechnet werden. Hierzu können im Rahmen des Verfahrens entsprechende Referenzwerte, Vergleichstabellen oder sonstige Bezugsdaten hinterlegt sein, gegen die die aufgenommenen Fotoaufnahmen und/oder Abfrageparameter abgeglichen werden. Auf dieser Grundlage kann dem Patienten die Selbstdiagnose erleichtert werden.

Bei der zur Auswertung fotografierten Drainagelösung kann es sich vorliegend um die aus dem Bauchraum eines Patienten austretende Drainagelösung handeln, die im Falle einer Peritonitis getrübt sein kann und hierdurch dem Patienten Aufschluss über das Vorliegen einer Peritonitis geben kann.

Erfindungsgemäß wird mit dem Prinzip einer Streulichtmessung eine Fotoaufnahme durchgeführt, indem ein Helligkeitswert insbesondere über den gesamten Pixelbereich oder über einen Teilbereich von diesem der Kamera des Smartphones gemittelt berechnet wird. Dieser gemittelte Wert kann in Bezug zu einem Referenzwert oder Schwellenwert gesetzt werden und so dem Patienten das Feststellen einer Peritonitis erleichtert werden. Es ist auch alternativ oder zusätzlich denkbar, dass die Fotoaufnahme und/oder die Abfrageparameter verfahrensgemäß mittels des Smartphones an einen sachkundigen Arzt oder Pfleger versendet werden und das Ergebnis der von dem Arzt oder Pfleger vorgenommenen Bewertung der Fotoaufnahme bzw. der Abfrageparameter wiederum verfahrensgemäß dem Nutzer des Verfahrens mitgeteilt wird.

In einer weiteren bevorzugten Ausführung ist denkbar, dass die Streulichtmessung in Reflexion erfolgt. Hierzu können eine Lichtquelle und die Kamera des Smartphones direkt benachbart am Smartphone vorgesehen sein, so dass bei entsprechender Beleuchtung der Katheteraustrittsstelle, der Drainagelösung bzw. des Drainagebeutels oder -schlauchs das von dem beleuchteten Objekt reflektierte Streulicht erfasst und ausgewertet werden kann.

In einer weiteren bevorzugten Ausführung ist denkbar, dass verfahrensgemäß eine Bilderkennung der Fotoaufnahme durchgeführt wird. Hierzu kann ein Foto mittels der Fotokamera mit oder ohne LED- bzw. Blitzlicht aufgenommen werden und es können daraufhin einzelne Bereiche des Bildes oder das gesamte Bild mit einer Bilderkennung ausgewertet und bspw. mit Referenzbildern verglichen werden. Bei der Bilderkennung können bestimmte Farb- oder Helligkeitswerte des Fotos mit vorgegebenen Parametern verglichen werden, wobei beispielsweise eine erkannte Trübung der Dialyselösung oder eine Rötung der Katheteraustrittsstelle dem Patienten besonders angezeigt werden können. Wie oben erwähnt ist auch ein alternatives oder zusätzliches Versenden der Fotoaufnahme zur Auswertung durch einen Arzt denkbar.

Gemäß der Erfindung ist die Fotoaufnahme eine Aufnahme der Drainagelösung innerhalb eines Schlauchs oder eines Beutels eines Peritonealdialysegeräts. Ein Benutzer bzw. Patient kann so während der selbständigen Durchführung einer Peritonealdialyse mittels eines Peritonealdialysegeräts einfacher und selbstständig die Drainagelösung auf eine ggf. vorhandene und häufig durch eine Trübung der Dialyseflüssigkeit angezeigte Peritonitis untersuchen. Es ist hierfür nicht notwendig, das Peritonealdialysegerät selbst umfassend zu modifizieren, sondern ein Nutzer kann sein eigenes Smartphone durch aufspielen einer entsprechend das Verfahren durchführenden Computersoftware zur Selbstdiagnose nutzen.

In einer weiteren bevorzugten Ausführung ist ferner denkbar, dass zur Auswertung der Fotoaufnahme der Drainagelösung wenigstens eine Markierung an dem Schlauch und/oder an dem Beutel verwendet wird. So können bspw. an der Unterseite des Beutels Markierungen ausgeführt sein, die in unterschiedlichen Strichstärken ausgeführt sein können. Das Smartphone kann dabei an der Oberseite des Beutels positioniert sein und die Unterseite durch den Beutel und durch die darin enthaltene Drainagelösung hindurch fotografiert werden. Der Grad der Sichtbarkeit der Markierungen kann mittels des erfindungsgemäßen Verfahrens automatisiert ausgewertet werden und als Maß für die Trübung der Drainagelösung dienen. Es können auch zusätzlich oder alternativ auf der Oberseite des Beutels Markierungen angebracht sein, die die Bilderkennung erleichtern und den Einfluss des Abstandes von Kamera zu Beutel sowie die Orientierung der Kamera relativ zum Beutel minimieren.

In einer weiteren bevorzugten Ausführung kann ferner vorgesehen sein, dass zwei Bereiche des Schlauchs mittels Fotoaufnahmen ausgewertet werden, wobei ein Bereich dem Zulauf und ein Bereich dem Ablauf eines Peritonealdialysegeräts entspricht. Bei dem Schlauch kann es sich dabei um einen einstückigen Schlauchabschnitt handeln oder es können auch zwei Teilschläuche den Zulauf und den Ablauf des Peritonealdialysegeräts bilden und erfindungsgemäß ausgewertet werden.

Weitere Vorteile und Einzelheiten der Erfindung sind anhand der in den Figuren beispielhaft gezeigten Ausführung erläutert. Dabei zeigen:

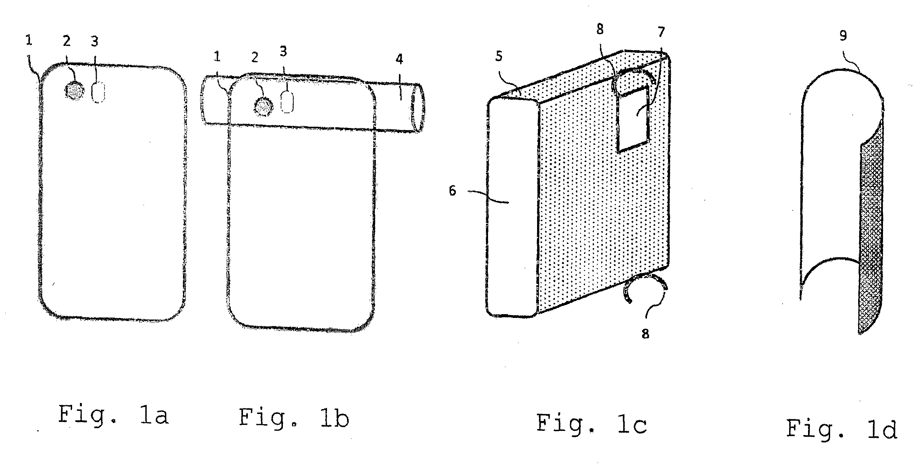

- Fig. 1a:: Rückansicht eines Smartphones;
- Fig. 1b:: Rückansicht eines Smartphones mit Schlauch;
- Fig. 1c:: Erfindungsgemäße Haltevorrichtung; und
- Fig. 1d:: Erfindungsgemäße lichtdichte Schlauchabdichtung.

Figur 1a zeigt ein Smartphone 1 mit einem Kameraobjektiv 2 bzw. mit einer Fotokamera 2, einem LED-/Blitzlicht 3 und einer auf der dem Kameraobjektiv 2 üblicherweise gegenüberliegenden Seite vorgesehen Eingabefläche bzw. einem entsprechenden Eingabebereich zum Eingeben von Informationen. Der Eingabebereich kann hierbei üblicherweise als Touchscreen ausgebildet sein.

Figur 1b zeigt das Smartphone 1 von Figur 1a mit einem dem Kameraobjektiv 2 vorgelagerten Schlauch 4, bei dem es sich um den Schlauch eines Peritonealdialysegeräts zur Leitung einer Drainagelösung handeln kann. Der Schlauch 4 ist hierbei direkt vor dem Kameraobjektiv 2 angeordnet und damit von der Fotokamera erfassbar. Insbesondere kann der Schlauch 4 so angeordnet sein, dass der gesamte von der Fotokamera 2 erfasste Bereich von dem Schlauch 4 ausgefüllt ist. Dies kann durch Koppeln des Schlauchs 4 und des Smartphones 1 mit einer Haltevorrichtung 5 erleichtert werden.

Figur 1c zeigt eine Vorrichtung zur Streulichtmessung am Schlauch 4 bzw. eine eben genannte Haltevorrichtung 5 zum Halten eines Smartphones 1. Das Smartphone 1 kann beispielsweise über eine Öffnung 6 zur Aufnahme des Smartphones 1 in die Haltevorrichtung 5 eingeführt werden. Denkbar ist aber auch eine Ausführung, bei der das Smartphone 1 nicht in die Haltevorrichtung 5 eingeführt wird sondern lediglich an dieser befestigt bzw. mit dieser gekoppelt wird. An der Haltevorrichtung 5 kann ein Fenster 7 vorgesehen sein, durch welches das Kameraobjektiv 2 und das LED-/Blitzlicht 3 hindurch auf einen davor bzw. daneben angeordneten Schlauch 4 gerichtet werden können. Der Schlauch 4 kann mittels einer Schlauchhalterung 8 im Bereich des Fensters 7 gehalten werden. Denkbar ist aber auch eine komplexere Ausgestaltung des Haltebereichs, bei dem zwei oder mehr Schläuche 4 oder Schlauchabschnitte im Bereich des Fensters 7 gehalten werden können.

Figur 1d zeigt eine lichtdichte Schlauchabdeckung 9, welche so mit der Haltevorrichtung 5 und/oder mit dem Schlauch 4 koppelbar ist, dass ein mittels des Smartphones 1 fotografierter Bereich des Schlauchs 4 gemäß der Erfindung abgedunkelt ist. Hierdurch kann für stabilere Lichtverhältnisse im abgebildeten Bereich und damit für bessere Auswertungen der Fotoaufnahmen gesorgt werden.

Bei der Ausführung der Erfindung sind die Messprinzipien der Streulichtmessung und der Bilderkennung möglich:
Bei der Streulichtmessung wird das Smartphone 1 mit seiner Kamera 2 direkt auf das Messobjekt, beispielsweise den Schlauch oder den Beutel gehalten. Zur Messung leuchtet die LED bzw. der Blitz 3 und deren Licht wird an der im Messobjekt befindlichen trüben Lösung gestreut und trifft auf die Kamera. Es wird ein Helligkeitswert bzw. Farbwert über den gesamte Pixelbereich der Kamera 2 gemittelt ausgewertet.

Der ermittelte Wert wird in Verhältnis zu einem Referenz- oder Schwellenwert gesetzt, der beispielsweise an dem Schlauchstück gemessen wird, das mit einer klaren Lösung, z.B. der zugeführten Peritonealdialyselösung gefüllt ist.

Die Licht- bzw. Farbintensität ist proportional zur Trübung/Färbung der Lösung. Da LED 3 und Kamera 2 bei einem Smartphone 1 direkt benachbart sind, kann die Streulichtmessung in Reflexion erfolgen.

Bei der Bilderkennung wird ein Foto (mit oder ohne LED/Blitzlicht 3) aufgenommen. Einzelne Bereiche des Bildes werden mit Bilderkennung ausgewertet.

Bei beiden Messprinzipien sind als Messorte der Schlauch oder der Beutel möglich Mögliche Ausführungsformen umfassen daher:
Eine Kombination von Streulichtmessung mit Messung am Schlauch, wobei es hierzu idealerweise eine Hilfsvorrichtung bzw. Haltevorrichtung 5 gibt, die mit Clips 8 bzw. Schlauchhaltebereichen 8 am Schlauch 4 eine fixe Position des Smartphones 1 zum Schlauch 4 sicherstellt. Außerdem verfügt das Hilfsmittel 5 über eine Aufnahmeöffnung 6, durch die das Smartphone 1 gesteckt wird, wodurch es am Schlauch 4 so fixiert werden kann, dass LED 3 und Kamera 2 direkt am Schlauch 4 anliegen. Die Vorrichtung hat ein Fenster 7, das für LED 3 und Kamera 2 einen Lichtdurchtritt zum Schlauch 4 zulässt. Die Hilfsvorrichtung 5 ist aus einem lichtundurchlässigen Material und schirmt Fremdlicht von der Messstrecke ab. Zur Abschirmung des Schlauches 4 von Fremdlicht im Bereich der Messstrecke und darüber hinaus dient eine Schlauchabdeckung 9.

Vor der Messung an der auslaufenden Drainage kann eine Kalibrierung am leeren Schlauch 4 erfolgen.

Denkbar ist auch eine Kombination von Bilderkennung mit einer Messung bzw. Fotoaufnahme am Beutel. Der Patient fotografiert dabei den Beutel nach vollständiger Drainage. Zur Auswertung gibt es folgende Optionen:
Zum einen kann ein automatisierter Vergleich des Bildes mit einer Bibliothek von Bildern durchgeführt werden. Zum anderen können sich auf der Unterseite des Beutels Markierungen befinden, ggf. in unterschiedlichen Strichstärken. Der Grad der Sichtbarkeit der Markierungen wird automatisch ausgewertet und gilt als Maß für die Trübung. Zusätzlich können auf der Oberseite des Beutels ebenfalls Markierungen angebracht sein, die die Bilderkennung erleichtern und den Einfluss des Abstandes von Kamera 2 zu Beutel sowie Orientierung von Kamera 2 relativ zum Beutel minimieren. Außerdem könnte die Trübung durch den Unterschied der Schärfe/Helligkeit zwischen Markierung auf Ober- und Unterseite ausgewertet werden.

Eine weitere Maßnahme kann das Anbringen eines weißen Feldes zum Weißableich sein, um Farbinformationen auswerten zu können, um beispielsweise eine Farbänderung des Dialysats zu erfassen.

Es ist ferner eine Kombination von Streulichtmessung mit einer Messung am Beutel denkbar. Wegen der Flexibilität des Beutels ist ein direktes Auflegen des Smartphones 1 auf den Beutel aber nicht so gut reproduzierbar wie das Anbringen am Schlauch 4. Außerdem ist die Abschirmung von Fremdlicht wegen der Größe und Beschaffenheit des Beutels schwieriger.

Weiterhin ist eine Kombination von Bilderkennung mit einer Messung am Schlauch denkbar. Dabei kann das Smartphone 1 an einem Punkt fixiert werden, an dem es sowohl den Zulauf wie auch den Ablauf erfasst. Der Zulauf kann dabei als Referenzwert dienen und die Trübung des Ablaufs relativ dazu gemessen werden.

Zur Absicherung des Verdachts auf eine Peritonitis sind folgende Kombinationen möglich:
Eine Trendanalyse des Grades der Trübung, wobei ein Ansteigen der Trübung über aufeinanderfolgende Beutelwechsel ein stärkeres Indiz für eine Peritonitis ist.

Eine Kombination von einer Messung der Trübung mit Abfragen von Symptomen des Patienten.

Ein Abgleich des Streulichtgrades bzw. der Trübungsstärke mit einem Schwellwert oder Übergangsbereich, z. B. kann es ein Histogramm von Werten bei Patienten ohne Peritonitis geben, und ein Histogramm von Patienten mit Peritonitis. Beide Histogramme dürften sich überlappen. Im Überlappungsbereich lägen dann der Übergangsbereich, bzw. ein fester Schwellwert.

Symptome wie Unterleibsschmerzen, Übelkeit, Anstieg der Körpertemperatur bzw. Gefühl von Fieber, Verstopfung und Durchfall können durch das Smartphone 1 bzw. durch das Computerprogramm oder Verfahren regelmäßig abgefragt werden, um einen initialen Verdacht auf Peritonitis zu erfassen. Diese Symptome können somit die erfindungsgemäßen Abfrageparameter darstellen. Erfindungsgemäß kann dabei ein einzelnes oder eine Kombination der genannten Symptome abgefragt werden.

Außerdem kann ad hoc bei Verdacht auf eine Peritonitis durch eine positive Trübungsmessung die Symptomabfrage zur Absicherung des Verdachts durchgeführt werden.

Es kann ferner vorgesehen sein, dass mittels eines Computerprogramms die Katheteraustrittsstelle des Peritonealdialysegeräts beispielsweise bei begründetem Verdacht des Patienten fotografiert und ausgewertet wird. Die Fotoaufnahme der Katheteraustrittsstelle kann hierbei automatisch über einen Bildvergleich mit hinterlegten Bildern ausgewertet werden und alternativ oder zusätzlich als telemedizinische Anwendung an einen betreuenden Arzt oder Pfleger zur Auswertung geschickt werden. Hierbei können insbesondere Exit Site Infektionen erkannt werden. Eine Exit Site Infektion ist üblicherweise gekennzeichnet durch eine Rötung, Schwellung und einen Druckschmerz im Bereich der Katheteraustrittsstelle. Diese Infektionen können nur oberflächlich vorhanden sein oder auch sich auch in subkutane Bereiche erstrecken.

## Patentansprüche

1. Verfahren zur Erfassung von Symptomen einer Peritonitis, wobei das Verfahren folgende Schritte umfasst:
• Aufnahme eines Fotos einer Drainagelösung und/oder einer Katheteraustrittsstelle durch ein Smartphone (1) und Eingabe wenigstens eines Abfrageparameters, der von einem Patienten über den Eingabebereich eines Smartphones eingegeben wird,
• Erfassung von Symptomen einer Peritonitis mittels Auswertung des Fotos und des Abfrageparameters, wobei die Fotoaufnahme eine Aufnahme eines Schlauchs (4) zur Leitung einer Drainagelösung ist, und mit dem Prinzip einer Streulichtmessung eine Fotoaufnahme durchgeführt wird;
**gekennzeichnet dadurch, dass**:
ein mittels des Smartphones (1) fotografierter Bereich des Schlauch: (4) mittels einer lichtdichten Schlauchabdeckung (9) lichtgeschützt bzw. abgedunkelt ist, und
die Fotoaufnahme durchgeführt wird, indem ein Helligkeitswert insbesondere über den gesamten Pixelbereich oder über einen Teil des gesamten Pixelbereichs der Kamera (2) des Smartphones (1) gemittelt berechnet werden.

2. Verfahren nach Anspruch 1, **dadurch gekennzeichnet, dass** der berechnete Wert in Verhältnis zu einem Referenz- oder Schwellenwert gesetzt wird, der beispielsweise an dem Schlauchstück gemessen wird, das mit einer klaren Lösung, insbesondere mit der dem Patienten zugeführten Peritonealdialyselösung gefüllt ist.

3. Verfahren nach Anspruch 1 oder 2, **dadurch gekennzeichnet, dass** die Streulichtmessung in Reflexion erfolgt.

4. Verfahren nach einem der vorhergehenden Ansprüche, **dadurch gekennzeichnet, dass** eine Bilderkennung der Fotoaufnahmen durchgeführt wird.

5. System zur Erfassung von Symptomen einer Peritonitis, umfassend ein Smartphone und eine Haltevorrichtung für das Smartphone mit einem Haltebereich zum Halten des Smartphones, einem Schlauchhaltebereich zum Halten eines Schlauchs und einem Fenster, welches dazu ausgebildet ist, das Fotografieren eines im Schlauchhaltebereich gehaltenen Schlauchs mittels des vom Haltebereich gehaltenen Smartphones zu ermöglichen,
**gekennzeichnet dadurch,**
**dass** eine lichtdichte Schlauchabdeckung vorgesehen ist, welche so mit der Haltevorrichtung koppelbar ist, dass ein mittels des Smartphones fotografierter Bereich abgedunkelt ist; und
**dass** das Smartphone ein Computerprogramm und einen Prozessor zur Durchführung des Verfahrens nach einem der Ansprüche 1-4 umfasst.

## Claims

1. Method of detecting symptoms of peritonitis, wherein the method comprises the following steps:
- taking a photo of a drainage solution and/or of a catheter exit site using a smartphone (1) and/or inputting at least one query parameter which is input by a patient through the input zone of a smartphone; and,
- detecting symptoms of a peritontitis by evaluating the photo and the query parameter, wherein
the photograph is a photo of a hose (4) for conducting a drainage solution, and the photograph is taken by means of scattered light measurement; **characterized in that**:
a region of the hose (4) photographed via the smartphone (1) is protected from light, or shaded, by means of an lightproof hose cover (9), and
the photograph is taken by calculating in an averaged manner a brightness value in particular over the total pixel range or over a part of the total pixel range of the camera (2) of the smartphone (1).

2. Method in accordance with claim 1, **characterized in that** the calculated value is correlated with a reference value or threshold value which is, for example, measured at the hose piece which is filled with a clear solution, in particular with the peritoneal dialysis solution supplied to the patient.

3. Method in accordance with claim 1 or claim 2, **characterized in that** the scattered light measurement takes place in reflection.

4. Method in accordance with any one of the preceding claims, **characterized in that** an image recognition of the photos is carried out.

5. System for detecting peritontitis, comprising a smartphone and a holder for the smartphone having a holding region for holding the smartphone, a hose holding region for holding a hose, and a window which is configured to enable the photographing of a hose held in the hose holding region by means the smartphone held by the holding region,
**characterized in that**
lightproof hose cover is provided which can be coupled to the holding apparatus such that a region photographed by means of the smartphone is shaded; and that the smartphone comprises a computer program and a processor for performing the method in accordance with one of claims 1 to 4.

## Revendications

1. Procédé pour détecter des symptômes d'une péritonite, dans lequel le procédé comprend des étapes suivantes :
- la prise d'une photo d'une solution de drainage et/ou d'un point de sortie de cathéter par un téléphone intelligent (1) et la saisie d'au moins un paramètre d'examen qui est saisi par un patient par l'intermédiaire de la zone de saisie d'un téléphone intelligent,
- la détection de symptômes d'une péritonite au moyen de l'évaluation de la photo et du paramètre d'examen, dans lequel
la prise de photo est une prise d'un tube flexible (4) pour l'acheminement d'une solution de drainage, et une prise de photo est mise en œuvre avec le principe d'une mesure de lumière diffusée ;
**caractérisé en ce que** :
une zone photographiée du tube flexible (4) au moyen du téléphone intelligent (1) est protégée de la lumière ou est obscurcie au moyen d'un recouvrement de tube flexible (9) opaque, et
la prise de photo est mise en œuvre **en ce qu'**une valeur de clarté est calculée de manière pondérée en particulier sur la totalité de la zone de pixels ou sur une partie de la totalité de la zone de pixels de la caméra (2) du téléphone intelligent (1).

2. Procédé selon la revendication 1, **caractérisé en ce que** la valeur calculée est mise en rapport avec une valeur de référence ou de seuil, qui est mesurée par exemple sur le tronçon de tube flexible, qui est rempli d'une solution claire, en particulier de la solution de dialyse péritonéale amenée au patient.

3. Procédé selon la revendication 1 ou 2, **caractérisé en ce que** la mesure de lumière diffusée est effectuée en réflexion.

4. Procédé selon l'une quelconque des revendications précédentes, **caractérisé en ce qu'**une identification d'image des prises de photo est mise en œuvre.

5. Système pour détecter des symptômes d'une péritonite, comprenant un téléphone intelligent et un dispositif de maintien pour le téléphone intelligent, avec une zone de maintien pour maintenir le téléphone intelligent, une zone de maintien de tube flexible pour maintenir un tube flexible et une fenêtre, laquelle est réalisée pour permettre de photographier un tube flexible maintenu dans la zone de maintien de tube flexible au moyen du téléphone intelligent maintenu par la zone de maintien,
**caractérisé en ce**
**qu'**un recouvrement de tube flexible opaque est prévu, lequel peut être couplé au dispositif de maintien de telle sorte qu'une zone photographiée au moyen du téléphone intelligent est obscurcie ; et
**que** le téléphone intelligent comprend un programme informatique et un processeur pour la mise en œuvre du procédé selon l'une quelconque des revendications 1 - 4.
